# EUROPEAN PATENT APPLICATION

(11) **EP 4 099 341 A1**
(43) Date of publication of application: **07.12.2022**
(21) Application number: 22176122.4
(22) Date of filing: 30.05.2022
(51) Int. Cl.: G16H 50/20, G16H 50/30, A61B 5/024, A61B 10/00, A61B 5/00

(54) **MENSTRUAL CYCLE TRACKING AND PREDICTION**

(30) Priority: 06.06.2021 US 202163197428 P; 16.05.2022 US 202217745548
(71) Applicant: Apple Inc., Cupertino, CA 95014 (US)
(72) Inventor: ZHANG, Shunan, Cupertino, 95014 (US); ZHANG, Carey Y., Cupertino, 95014 (US); PARK, Jihyun, Cupertino, 95014 (US); CHAN, Vincent M., Cupertino, 95014 (US); BRITTAIN, Roxanne B., Cupertino, 95014 (US); REN, You, Cupertino, 95014 (US); WANG, Yikai, Cupertino, 95014 (US)
(74) Representative: COPA Copenhagen Patents

(57) **Abstract**

A mechanism for estimating windows for menstrual cycles and fertility. The mechanism tracks menstrual cycles based on accuracy of predictions as compared to user input logging the start and stop of a cycle; this user input and the accuracy of prior cycle predictions is used to improve and update future predictions. Thus, as the mechanism receives additional data, it refines its predicted fertility window and period. A user's heart rate may be used to estimate fertility windows, periods, and other portions of a menstrual cycle.

## Description

### CROSS-REFERENCE TO RELATED APPLICATIONS

This application claims the benefit under 35 U.S.C. § 119(e) of U.S. Provisional Patent Application No. 63/197,428, filed June 6, 2021, the contents of which are incorporated herein by reference in their entirety.

### FIELD

The described embodiments relate generally to menstrual cycle tracking and prediction. More particularly, the present embodiments relate to estimating a fertility window and period date based on biometric information of a user, such as a heart rate.

### BACKGROUND

Many people that experience menstrual cycles plan activities around portions of their menstrual cycle, such as a fertility window and/or period. Menstrual cycles can be irregular, leading to an inability to reliably plan activities. Further, attempts to gather data that may be used to estimate or predict portions of menstrual cycle may be difficult, invasive, and inaccurate.

### SUMMARY

Embodiments described herein generally take the form of a mechanism for estimating dates of various portions of a menstrual cycle, such as a period, fertility window, and/or ovulation. The mechanism tracks these portions of menstrual cycles based on accuracy of predictions as compared to user input logging the start and stop of a cycle; this user input and the accuracy of prior cycle predictions is used to improve and update future predictions. Thus, as the mechanism receives additional data, it refines its predicted fertility window and period.

A "menstrual cycle" lasts from the start of a period to the day before the start of the next period and typically includes a follicular phase, ovulation, and a luteal phase. A "fertility window" is the portion of the menstrual cycle during which a user is most likely to conceive; it typically begins several days (e.g., up to five days) before ovulation and typically ends shortly (e.g., one day) after ovulation. The follicular phase occurs after the period and prior to ovulation and the luteal phase occurs post-ovulation, extending to the start of the next period.

Generally, updated period and/or fertility estimates are provided to a user only under certain circumstances rather than routinely upon determination, or whenever the estimates are otherwise updated. Put another way, even though an embodiment may calculate or provide various fertility window and/or period estimates, it may not necessarily display those estimates to a user. For example, updated estimates may be provided only when a probability of such an estimate being accurate exceeds a threshold (e.g., has a high confidence score). As another option, such estimates or updates may be provided only when the cycle or fertility windows start or stop more than a certain number of days from the present date. As a more specific example, some embodiments may only update an estimate for a current window (whether menstrual or fertility) if a close of the window is more than a predetermined number of days (e.g., two days or three days) away, insofar as updating a window having a shorter time to close may limit planning capabilities for a user. Further, certain embodiments may update estimated dates only for a next period or fertility window rather than all future windows, thereby reducing a number of updates and/or a chance of compounding errors in a series of such estimates.

In some embodiments, a user may receive an alert when an updated period and/or fertility estimate is provided to a user. In an example of these embodiments, user alerts associated with updated estimates for a cycle's start, stop, and or duration, as well as the same for fertility, may be limited to a predetermined number within any given time period. Each type of updated estimate (a fertility estimate and a period estimate) may have its own limit alert limit, or there may be a limit to the overall number of alerts regardless of the type of updated estimate. For example, a user may receive a maximum of one alert associated with an updated period estimate each month and a maximum of one alert associated with an updated fertility estimate each month.

An embodiment may track a heart rate of a user across a period of time, both while the user is awake and while the user is asleep. Certain changes in the heart rate of a user, and particularly the user's sedentary heart rate, occur when a user is fertile; typically, the sedentary or resting heart rate of a person increases by approximately two beats per minute during the fertility window. For many people, the sedentary heart rate increases during the ovulatory and luteal phases as compared to the menstruation and follicular phases. Thus, detecting an increased sedentary heart rate of a user may indicate that a period has begun or is about to begin. By correlating such heart rate increases (which typically occur for an extended period of time, such as a six-hour monitoring period, a day, or the like) to current estimations of menstrual and/or fertility windows, the accuracy of these estimations may be assessed and, if necessary, the estimated dates of the windows' opening and closing may be updated as discussed above.

One embodiment takes the form of a wearable device for estimating portions of a menstrual cycle, comprising: a calendar module; a heart rate sensor; a preprocessing module operative to: receive an initial period estimate from the calendar; receive heart rate data from the heart rate sensor; and process the initial period estimate and heart rate data into a processed data set; an ovulation estimator operative to: receive the processed data set from the preprocessing module; and use the processed data set to estimate a fertility window; and a period estimator operative to: receive the processed data set and an output from the ovulation estimator; and use the processed data set and the output from the ovulation estimator to estimate a period date.

Another embodiment takes the form of a method for providing estimates of a fertile window, comprising: receiving an initial estimate of a fertile window and an initial estimate of a period; receiving heart rate data; determining whether the heart rate data covers a sufficient period of time; in the event the heart rate data covers the sufficient period of time, estimating an ovulation window using the heart rate data and the initial estimate of the fertile window; and in response to estimating the fertile window, updating a display of the fertile window that is electronically accessible by a user.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows a sample graph of a sedentary heart rate during a menstrual cycle.
FIG. 2 shows a set of modules of an embodiment of a menstrual cycle estimator.
FIG. 3 shows a sample user interface displaying estimated dates for a period, including less likely and more likely dates of the period.
FIG. 4 is a flowchart showing a sample method for estimating probable dates of a fertility window and period from a user's biometric data, such as a heart rate.
FIG. 5 illustrates a sample wearable device that may estimate probable dates of a fertility window and period.
FIG. 6 illustrates sample components of the wearable device of FIG. 5.

### DETAILED DESCRIPTION

Reference will now be made in detail to representative embodiments illustrated in the accompanying drawings. It should be understood that the following descriptions are not intended to limit the embodiments to one preferred embodiment. To the contrary, it is intended to cover alternatives, modifications, and equivalents as can be included within the spirit and scope of the described embodiments as defined by the appended claims.

Embodiments described herein generally take the form of a mechanism for estimating dates of various portions of a menstrual cycle, such as a period, fertility window, and/or ovulation. The mechanism tracks these portions of menstrual cycles based on accuracy of predictions as compared to user input logging the start and stop of a cycle; this user input and the accuracy of prior cycle predictions is used to improve and update future predictions. Thus, as the mechanism receives additional data, it refines its predicted fertility window and period.

A "menstrual cycle" lasts from the start of a period to the day before the start of the next period and typically includes a follicular phase, ovulation, and a luteal phase. A "fertility window" is the portion of the menstrual cycle during which a person is most likely to conceive; it typically begins several days (e.g., up to five days) before ovulation and typically ends shortly (e.g., one day) after ovulation. The follicular phase occurs after the period and prior to ovulation and the luteal phase occurs post-ovulation, extending to the start of the next period.

Generally, updated period and/or fertility estimates are provided to a user only under certain circumstances rather than routinely upon determination, or whenever the estimates are otherwise updated. Put another way, even though an embodiment may calculate or provide various fertility window and/or period estimates, it may not necessarily display those estimates to a user. For example, updated estimates may be provided only when a probability of such an estimate being accurate exceeds a threshold (e.g., has a high confidence score). As another option, such estimates or updates may be provided only when the period or fertility windows start or stop more than a certain number of days from the present date. As a more specific example, some embodiments may only update an estimate for a current window (whether period or fertility) if a close of the window is more than a predetermined number of days (e.g., two days or three days) away, insofar as updating a window having a shorter time to close may limit planning capabilities for a user. Further, certain embodiments may update estimated dates only for a next period or fertility window rather than all future windows, thereby reducing a number of updates and/or a chance of compounding errors in a series of such estimates.

In some embodiments, a user may receive an alert when an updated period and/or fertility estimate is provided to a user. In an example of these embodiments, user alerts associated with updated estimates for a cycle's start, stop, and or duration, as well as the same for fertility, may be limited to a predetermined number within any given time period. Each type of updated estimate (a fertility estimate and a period estimate) may have its own limit alert limit, or there may be a limit to the overall number of alerts regardless of the type of updated estimate. For example, a user may receive a maximum of one alert associated with an updated period estimate each month and a maximum of one alert associated with an updated fertility estimate each month.

An embodiment may track a heart rate of a user across a period of time, both while the user is awake and while the user is asleep. Certain changes in the heart rate of a user, and particularly the user's sedentary heart rate, occur when a user is fertile; typically, the sedentary or resting heart rate of a person increases by approximately two beats per minute during the fertility window. For many people, the sedentary heart rate increases through the fertility window, during ovulation and into the luteal phase, then drops during the menstruation (e.g., period) and follicular phases. Thus, detecting an increased sedentary heart rate of a user may indicate that a fertility window has begun or is about to begin. By correlating such heart rate increases (which typically are measured for an extended period of time, such as a six-hour monitoring period, a day, or the like) to current estimations of periods and/or fertility windows, the accuracy of these estimations may be assessed and, if necessary, the estimated dates of the windows' opening and closing may be updated as discussed above.

Given the desirability of tracking sedentary heart rate at multiple times while a person is awake and asleep, some embodiments may take the form of a wearable device that incorporates a heart rate monitor. The wearable device may be, for example, an electronic watch with a heart rate sensor. The electronic watch may likewise accept user input to confirm or refine an accuracy of the estimated windows, for example through a touch-sensitive input, button, rotating input, or the like. Further, the electronic watch may incorporate a display configured to show graphical information related to the estimated windows. It should be appreciated that other embodiments need not be wearable devices; they may be various computing devices such as smartphones, tablet devices, laptop or desktop computers, and so on. Such embodiments may incorporate, or may be in communication with, a sensor (e.g., that is part of a wearable device) configured to measure a user's heart rate or other biometric information as discussed herein.

In particular, some embodiments may present graphical information that shows a range of likely dates for the start and end of a period and/or fertility window. As one option, a range of likely start and/or end dates may be shown, with the graphical information conveying a probability or likelihood that each such date is the start or end of the window(s). As one non-limiting example, dates may be color-coded to represent the probability that the date falls within the window in question. As estimates are updated or otherwise when appropriate, the color-coding of the dates may be likewise updated.

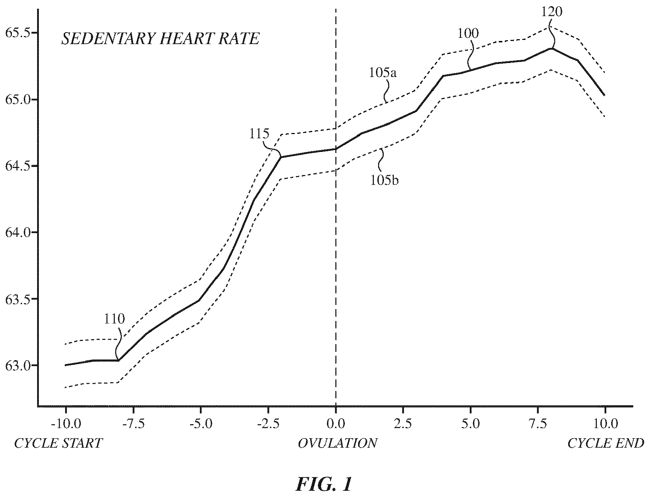

These and other embodiments are discussed below with reference to FIGs. 1-6. However, those skilled in the art will readily appreciate that the detailed description given herein with respect to these Figures is for explanatory purposes only and should not be construed as limiting.

Generally and as broadly discussed above, embodiments described herein track and predict future user fertility windows and periods. Menstrual cycles are tracked from a "day one" instantiation, which is the first day that a user initiates tracking by marking a first day of menstrual bleeding. On day one, a next fertility window and/or period is predicted for a user, optionally including a fertile window and/or ovulation date. As embodiments ingest additional data and refine that data through, for example, a neural network configured to learn from the data, more accurate predictions of periods, fertile windows, and/or ovulation dates are provided. Generally, embodiments can update fertile window and/or next period predictions mid-cycle based on ingested data.

Certain embodiments may incorporate sensor data to better estimate fertility window and/or period beginnings or endings. For example, an embodiment may take the form of a portable, wearable device that has one or more heart rate sensors. (In some embodiments, a temperature sensor may be used in addition to, or instead of, a heart rate sensor.) As shown in FIG. 1, a user's sedentary heart rate typically increases shortly before the user's luteal phase begins and rises steadily through ovulation, peaking shortly after ovulation around a beginning of the follicular phase. While the heart rate and its relationship to the menstrual cycle as shown in FIG. 1 is but an example, most people have a similar correlation between sedentary heart rate and various portions of the menstrual cycle. This is especially true if the user's sedentary heart rate is gathered and averaged, smoothed, or otherwise algorithmically correlated across multiple menstrual cycles. Thus, a user's sedentary heart rate may be used to predict various portions of a menstrual cycle, or to refine predictions of such portions of a menstrual cycle, such as the onset and end of a fertility window, a period, and/or ovulation. As one non-limiting example, increases in a person's heart rate often start at, near, or shortly after the opening of the fertility window.

For example and as shown in FIG. 1, in the follicular cycle a person's sedentary (e.g., resting) heart rate 100 begins to climb. This is represented by the breakpoint 110, which in this example occurs approximately eight days before ovulation. The person's resting heart rate continues to climb, often (though not necessarily) in a fairly steady fashion, through much of the follicular phase of the menstrual cycle.

As the person approaches ovulation, the rate of change of the heart rate generally decreases, although the heart rate itself continues to increase. Thus, at breakpoint 115, the slope of the heart rate vs. time changes. In the example of FIG. 1, this occurs approximately two days before ovulation. The fertility window often opens during this increase in heart rate.

In many cases, the rise in heart rate for a person during the follicular phase (which stretches from cycle start to ovulation) is fairly sharp and may be, for many people, one-and-a-half to two beats per minute. For most people the follicular phase is less regular than the luteal phase of menstruation, and so tracking changes in heart rate (and the rate of change of a heart rate) may provide the ability to accurately track a duration of the follicular phase as well as predict an onset of ovulation.

Likewise, heart rate typically continues to rise after ovulation through the luteal phase. The rate of change (e.g., slope) of this heart rate increase is fairly constant as measured across the luteal phase, and typically less than the rate of change in the follicular phase. Towards an end of the luteal phase most people's resting heart rates decline, thus providing a strong signal that an end of the luteal phase is approaching. Breakpoint 120 signals this decline in sedentary heart rate. The duration of the luteal phase is more consistent from cycle to cycle in most people, so accurately measuring one (or a series) of luteal phases enhances the ability to predict a start, stop, and length of future luteal phases, and thus dates of the fertility window, period, and/or ovulation.

It should be appreciated that the heart rate 100 shown in FIG. 1 is but one generalized example and is not representative of any particular heart rate or person. Further, even in the generalized example of FIG. 1, a user's heart rate 100 may vary from cycle to cycle. Thus, embodiments may use a range of heart rates rather than an actual or measured heart rate. The range may be determined by averaging heart rates across multiple cycles, by applying a distribution function or other process (such as an estimator or probabilistic analysis) to a measured heart rate 100, or the like to determine upper and lower bounds 115a, 115b in which a user's heart rate is expected to fall during any given cycle. These generalized ranges may be used by any embodiment, module, process, method, or the like described herein instead of, or with, a specific heart rate.

By measuring sedentary heart rate, for example through periodic sampling while wearing a wearable device incorporating a heart rate monitor, changes in heart rate (as well as the slope or rate of such changes) may be determined and used to predict various facets of future menstrual cycles, including possible or likely start dates and end dates of fertility windows and periods, as well as the same for ovulation. This information may be provided to a user through a calendar or other application executed by a computing device so that the user may plan future activities accordingly. This information may be used to plan attempts to become pregnant or, conversely, for contraception, for travelling, and so on. Accordingly, there is great value in accurately predicting a start and stop of various portions of a menstrual cycle, including a period and a fertility window.

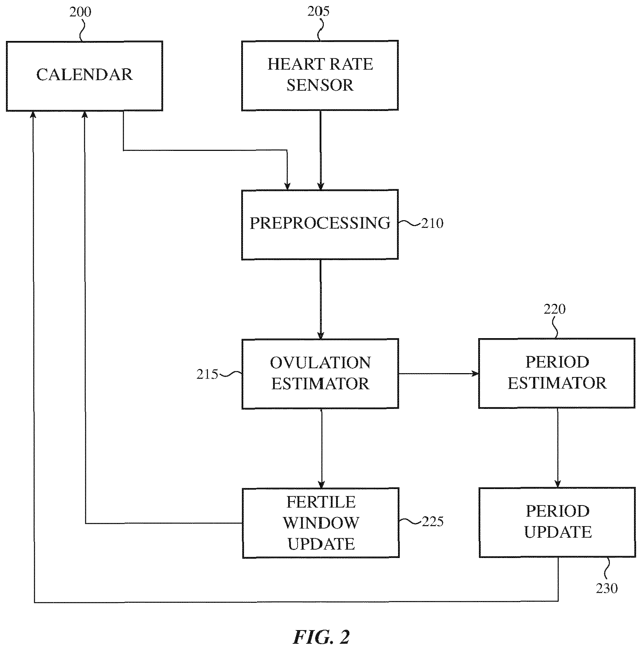

As mentioned, certain electronic devices, including certain wearable devices, may be configured to both detect a user's heart rate as well as employ that data to estimate start dates, end dates, and durations of both periods and fertility windows. FIG. 2 generally illustrates sample modules that may operate on, or be executed by, such an electronic device.

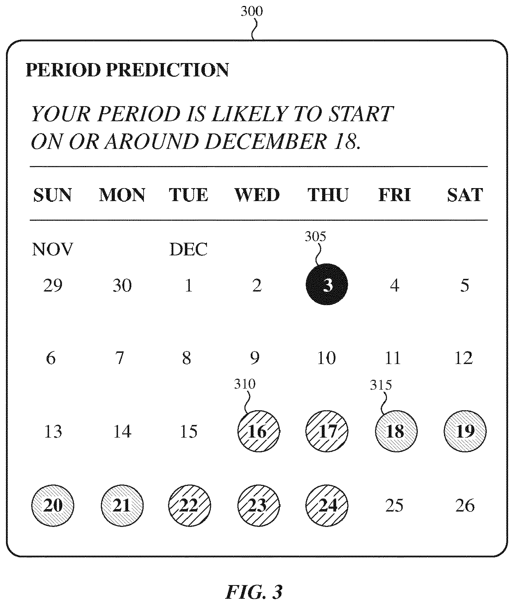

The sample electronic device may include a number of modules 200-225 that cooperate to provide and/or update estimates 230, 235 of both a fertile window and a period. For example, the modules may include a calendar module 200 that maintains information relating to a user's menstrual cycles. The calendar module 200 may contain user-inputted data and/or estimates relating to past menstrual cycles, a current menstrual cycle, and/or future menstrual cycles. Information from the calendar module 200 may be used to generate a user interface that depicting information about the user's menstrual cycle, such as discussed below with respect to FIG. 3C.

In certain embodiments, for a given menstrual cycle the calendar module 200 may provide an initial (or "day zero") estimate of either or both of a fertile window and a period. More specifically, the calendar module 200 may provide a range of dates on which a period may start and end, classifying some of those dates as possible start or end dates and others as more likely start or end dates. The calendar module 200 may do the same for the fertile window, although in some embodiments the calendar may not provide less likely dates for the fertile window. In certain embodiments, the calendar provides an estimate of the first day of a menstrual cycle, a fertile window within the menstrual cycle, and an estimate of a next period within a menstrual cycle.

As discussed in more detail with respect to FIG. 3, the calendar module 200 may rely on user-submitted data to provide its day zero estimates and may base those estimates on a variety of factors, such as tracked or typical lengths between periods, of luteal phases, or the like. The day zero estimate, or any future estimates resulting from iterations of estimations as detailed with respect to this figure and FIG. 3, may be transmitted to a preprocessor 210 from the calendar 200.

The heart rate sensor 205 may periodically sample or otherwise measure a user's heart rate, for example at five-minute intervals. Certain embodiments may use any heart rate measurement while other embodiments may focus on, or employ, heart rates corresponding to particular user states. As one example, an embodiment may employ sedentary heart rates rather than all heart rates when estimating ovulation, fertility windows, and/or periods. As another example, an embodiment may employ basal sedentary heart rates during such estimations, where basal rates are the lowest 10% of all heart rates measured or the like.

As discussed in more detail with respect to FIG. 3, the heart rate sensor may measure the user's sedentary heart rate both while the user is awake and while the user is asleep. The device may determine whether a user is awake or asleep based on a time at which the heart rate sensor measures the sedentary heart rate (e.g., day vs. night), by assigning heart rates above a threshold to an "awake" user state and below the threshold to an "asleep" user state (insofar as resting heart rates are often lower when a user is asleep than awake), or through any other suitable method or manner of assigning heart rate measurements to one category or the other. The measured sedentary heart rates may be transmitted to the preprocessor 210.

While a threshold of a number of beats per minute is used in some embodiments to categorize heart rates as "awake" heart rates or "asleep" heart rates, other criteria may be used to categorize heart rates. In some instances, motion information associated with a heart rate (which may be determined by correlating heart rate measurements with accelerometer or other motion sensor measurements) may be used to categorize heart rates, such heart rates collected during periods of low user motion (i.e., below a threshold amount of motion) are categorized as "asleep" heart rates. In still other variations a time-based threshold is used to categorize heart rates, such that heart rates collected during certain times are "awake" heart rates and those collected during other times are "asleep" heart rates. It should be appreciated that multiple different types of thresholds may be used simultaneously. For example, a motion threshold may be used during a first time period, and a different motion threshold may be used a second time period, which may be used to impose stricter motion criteria during daytime hours (when a user is less likely to be sleeping).

The preprocessor 210 ingests the calendar data and heart rate data and places it in a form to be used by the ovulation estimator 215, which may be a processed data set. The preprocessor 210 may, for example, weight either the calendar data or heart rate data according to a set of criteria. It may likewise determine whether to use heart rate data at all. For example and as discussed below in more detail with respect to FIG. 3, the preprocessor 210 may only incorporate, ingest, or otherwise pass to the ovulation estimator 215 heart rate data if it receives at least a minimum number of days of heart rate data and/or a minimum number of samples of heart rate data for each day within a predetermined window of time. In some examples, the preprocessor 210 may require at least 23 days of heart rate data (each having a threshold amount of heart rate data) within the last 45 days. Put more broadly, the preprocessor may only utilize heart rate data if the number of days with such data in a given window (a sufficient period of time) exceeds a threshold; in some embodiments, this threshold is half. That is, the preprocessor 210 may define a window of days and then utilize heart rate data, or pass on heart rate data (whether raw, normalized, imputed, normalized and imputed, or otherwise processed) if the data exists for at least the threshold number of days within the window. The preprocessor 210 may determine whether the heart rate data covers a sufficient period of time prior to processing the initial period estimate and heart rate data into a processed data set.

The preprocessor 210 may transmit a normalized matrix of heart rate data to the ovulation estimator 215 along with the data received from the calendar module 200. Generally, the preprocessor puts all data into a common form that is utilized by the ovulation estimator 215, such as a preprocessed data set.

The ovulation estimator 215 likewise receives the calendar data, including any day zero predictions, from the calendar module 200. The ovulation estimator 205 may be a twolayer neural network, including an LSTM neural network and a dense neural network. Other embodiments may use different neural network and/or different numbers of neural networks in the ovulation estimator module 215. The ovulation estimator utilizes the calendar data and the heart rate data to estimate a set of possible start and end dates for a user's ovulation and thus a fertility window. The ovulation estimator may utilize the following data to estimate ovulation and a fertility window: 1) calendar data, including the day zero prediction or any other calendar-based period predictions; 2) the number of times for which a waking sedentary heart rate was captured by the heart rate sensor in any day for which such a heart rate was captured; 3) the number of times for which a sleeping sedentary heart rate was captured by the heart rate sensor in any day for which such a heart rate was captured; 4) the average (or median, or weighted) basal sedentary heart rate(s) while awake for any day on which waking heart rate data was captured; 5) the average (or median, or weighted) basal sedentary heart rate(s) while sleeping for any day on which sleeping heart rate data was captured; and/or 6) the days on which any of #2-#5, above, were captured. In some embodiments, a "basal" sedentary heart rate is defined as a heart rate below a threshold in a data set of heart rates for any given day. For example, any heart rate in the lowest 10% of captured heart rates, on any given day, may be considered a basal heart rate for that day. Various embodiments may use different thresholds for basal heart rates or may ignore basal heart rates entirely and simply use an average of all sedentary heart rates for any given day or other time period, or a data set of all such heart rates, and so on.

The ovulation estimator 215 outputs an estimate of how likely a set of future days is to be part of a fertile window. Generally, the ovulation estimator 215 uses the heart rate data and calendar data, after it is processed by the preprocessor 210, to make multiple determinations of the likelihood that any given future day (out to a limit) is part of the fertile window. Each of these determinations is called a "profile," and the ovulation estimator generates profiles for the current day and a number of preceding days (for example, two preceding days in some embodiments, although any number of preceding days may be used). Every profile includes a probability for how likely each of the set of future days is to be part of the fertile window. These probabilities are based on the calendar data and heart rate data for each of a set of days (for example 30, 40, or 45 days) preceding the day for which a given profile is generated. Thus, where the profile is generated for today, data for a preceding 45 days may be used to generate the profile. Where the profile is generated for yesterday, data for 45 days preceding yesterday may be used, and so on. It can be seen that the data used to generate any given profile is offset by one day from data used to generate profiles for adjacent days.

The ovulation estimator 215 compares the probabilities for each day in each profile to one another. That is, for a given day D, the ovulation estimator 215 compares the probability in profile 1 to the probability in profile 2, profile 3, and so on. If the probabilities that day D are relatively the same, or within a certain deviation, between each of the profiles, then day D is considered a likely part of the fertility window. As the probabilities between the profiles diverge, the likelihood of D being part of the fertility window decrease. Thus, by comparing the estimates in the various profiles to one another, the ovulation estimator 215 may build a reliable model of both future fertility windows and ovulation dates, as based on measured heart rates across a period of time, prior menstrual cycle data such as a prior period start and stop (as logged by a user with the calendar 205), and current calendar estimates of the fertility window and/or ovulation date.

The ovulation estimator 215 generally outputs each profile as a vector with a set of connected nodes, each node corresponding to a future day, and a probability for each node that it is part of the fertility window. In certain embodiments, each profile includes 25-40 nodes, and in some embodiments each profile includes 32 nodes. It should be appreciated that these numbers are but examples rather than limitations; any number of nodes may be present in each profile and each profile may include a different number of nodes from any other or all may include the same number of nodes. By using 32 nodes and three profiles, however, the ovulation estimator 215 may compare probabilities for every day across the next 30 days, thus giving approximately a one-month analysis of fertility window probabilities.

As the number of nodes in a profile increases, computational complexity and power used to generate each profile increases. Likewise, the further in the future any node is, the less accurate the estimates of probability for that node being part of the fertile window become. Thus, it may be practically useful to limit the number of nodes in a profile. Embodiments may so limit profiles to 30 nodes/30 days in order to provide relatively accurate estimates for the next month within a reasonable time.

The output of the ovulation estimator 215 is provided to two different modules, namely the period estimator 220 and the fertile window updater 225. The period estimator 215 will be discussed first.

The period estimator module 220, like the ovulation estimator module 215, is one or more neural networks cooperating with one another, such as an LSTM network and dense network. The period estimator 220 utilizes the output of the ovulation estimator 215 to determine whether or not an estimated period should be redetermined, changed, or otherwise updated. The period estimator 220 provides an updated estimate of a user's period if the ovulation estimator's 215 updated estimate for an ovulation date occurs in the past, that estimate is consistent for each of the present day and two preceding days, and the estimated ovulation date has a high degree of confidence (e.g., is a quality estimation). A "high degree of confidence" may be, for example, more than 50%, more than 60%, more than 75%, or otherwise above a predetermined threshold. It should be appreciated that other embodiments may omit any or all of these criteria when determining whether or not to update a period estimate, or may use different criteria instead of, or in conjunction with, these three.

The period estimator 220 generally operates similarly to the ovulation estimator 215, accepting the same inputs as the ovulation estimator. These inputs may come from the ovulation estimator 215 as shown in FIG. 2, or may directly come from the preprocessor 210. Presuming that ovulation has occurred and the luteal phase appears stable, which is determined by the criteria set out in the preceding paragraphs in some embodiments, the period estimator 220 calculates a profile in a manner similar to that described with respect to the ovulation estimator. Here, however, the profile is a vector with a set of nodes, each representing a future day, and including a likelihood of a period falling on a day corresponding to the node. As mentioned, calculating these profiles generally relies on the same inputs as used by the ovulation estimator 215.

The period estimator 220 profiles are passed to a period update module 230. The period update module determines whether or not to update estimates dates of a period from a current set of values, based on the probabilities determined by the period estimator 220. In one example, the period update module 230 updates an estimated date(s) of a period if the estimated period date changes and has not already been previously updated within a single menstrual cycle. Other embodiments may use different update criteria or may update every time the period estimator operates. The updated period estimate is passed from the period update module 230 to the calendar module 200, which may then be used to display a revised user interface with the new information and/or alert a user to this update. An example of this user interface is shown in FIG. 3 and discussed in more detail with respect to that figure.

As previously stated, the ovulation estimator 215 outputs its profiles to the fertile window update module 225 in addition to the period estimator 220. The fertile window update module 225 updates the estimated fertile window in accordance with the probabilities from the ovulation estimator 215. In one non-limiting example, the fertile window update module 225 will update the estimated fertile window presuming the newly-estimated fertile window: 1) has not yet started (e.g., is in the future); 2) starts within a maximum number of days, such as three, five, or the like (e.g., is not too far in the future); 3) has not yet been updated within the current menstrual cycle; 4) occurs after the currently-estimated fertile window; and 5) the dates of the newly-estimated fertile window have a high degree of confidence associated with them. The dates may have a "high degree of confidence" if they are more likely to be accurate than the dates of the currently-estimated fertile window, if they meet a threshold probability of being accurate (e.g., 50%, 60% 75%, and so on), or meet other predetermined criteria.

In the non-limiting example, presuming these criteria are met, the fertile window update module 225 provides the newly-estimated fertile window to the calendar module 200, which may then display a revised user interface with the new information. Again, this user interface is shown in FIG. 3. Some embodiments may use different criteria instead of, in addition to, or intermixed with any of the aforementioned criteria, or may simply update the fertile window without reference to any criteria at all.

FIG 3 is an example of a user interface showing a calendar 300 displaying dates of an estimated period. The dates of the estimated period may be received from the calendar module 200, and may utilize dates provided by the period estimator 220, as described with respect to FIG. 2.

As shown in FIG. 3, the current date 305 is highlighted on the calendar 300. Additionally, a set of estimated period dates are shown, with some corresponding to more likely period dates (e.g., date 315) and some to less likely period dates (e.g., date 310), where "more likely" and "less likely" are relative terms to address whether a given likelihood is above or below a given threshold. Typically, the more likely period dates 315 are bracketed by less likely period dates 310 in accordance with a probabilistic distribution generated by the profile estimator 220. Further, more likely period dates 315 may be visually differentiated from less likely period dates 310, for example by shading the dates, or icons associated with the dates, differently, by rendering them in different colors, and the like. As one example, a hue, intensity, or the like may be deeper or stronger for more probable dates and lighter or weaker for less probable dates. It should be appreciated that the criteria for updating period and/or fertility estimates may be the same or different as the criteria used to indicate probabilities of such events on certain days. For example, some embodiments may not update the days of an estimated fertility window or period as described above but may change the shading, color, or other indicator of probability for certain days. Thus, while the overall days in the fertility window or period may remain the same, the probability indicator for each such day may vary.

Although FIG. 3 illustrates a period on the calendar 300 with more and less likely probable dates, the same user interface and scheme may additionally or alternatively be used to display a fertility window, including more and less likely dates for the fertility window. Thus, outputs of the ovulation estimator 215 and/or fertile window update module 225 may be received by the calendar module 200 and may be shown by the calendar 300, as well.

Some embodiments may provide a user alert when a period or fertility window is updated. For example, an electronic device (whether the device calculating the estimates or another associated device) may provide an audible, visual, and/or haptic alert to a user to capture their attention and indicate the update has occurred. Certain embodiments may automatically open and display the calendar with the updated information, as another example. The frequency of these alerts may be limited in some embodiments, such as discussed above.

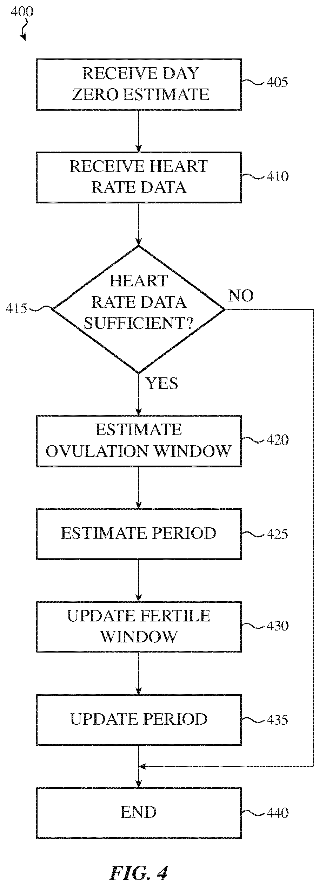

FIG. 4 is a flowchart illustrating a sample method 400 for estimating dates for a fertile window/ovulation and a period and updating a user interface, such as a calendar, with those estimates. It should be appreciated that this is but one sample method, and embodiments may use other methods, add operations, omit operations, or change the order of operations without departing from the spirit or scope of this disclosure. The method 400 begins in operation 405, in which the embodiment receives or generates a day zero estimate. The day zero estimate may have been previously generated, may be based on user-inputted information, or may be omitted entirely in various versions of this method and various embodiments. The day zero estimate may be provided by a calendar module 200 or otherwise from user input, as discussed with respect to FIG. 2.

In operation 410, the embodiment receives, records, retrieves, and/or generates heart rate data, for example through a heart rate sensor. As discussed above with respect to FIG. 2, the heart rate date for any given day may include a waking basal heart rate or rates, a sleeping basal heart rate or rates, a number of heart rate measurements while awake and/or asleep, and the like.

In operation 415, the embodiment determines whether the heart rate data is sufficient for use. This determination is discussed above with respect to FIG. 2 and may be performed by a preprocessor module 210 or other suitable software, hardware, or firmware. General suitability criteria are provided above. If the heart rate data is insufficient, then the method terminates in end state 440.

Presuming the heart rate data is sufficient, then in operation 420 the embodiment estimates the ovulation window for a user. The estimated ovulation window is typically a single date (an estimated "ovulation date" that represents the day during which ovulation is most likely to occur), but in other instances may include multiple dates that collectively represent the days during which ovulation is most likely to occur. The ovulation window may be predicted by an ovulation estimator 215 and details of the estimation are discussed with respect to FIG. 2.

Following operation 420, operations 425-430 may be executed in any order or simultaneously, so long as operation 435 follows operation 425. In operation 425, a period estimator 220 estimates likely dates of a period. In operation 430, the fertile window may be updated and the new dates provided to the calendar module 200 or a user interface module. Likewise, in operation 435 the period may be updated and new dates provided to the calendar module 200 or the like. Details regarding how and when the fertile window and period are updated are provided in the discussion of FIG. 2.

Finally, the method 400 ends in end state 440. If the method is executed again then the updated period and fertile windows provided in operations 430 and 435 form the new day zero estimate of operation 405.

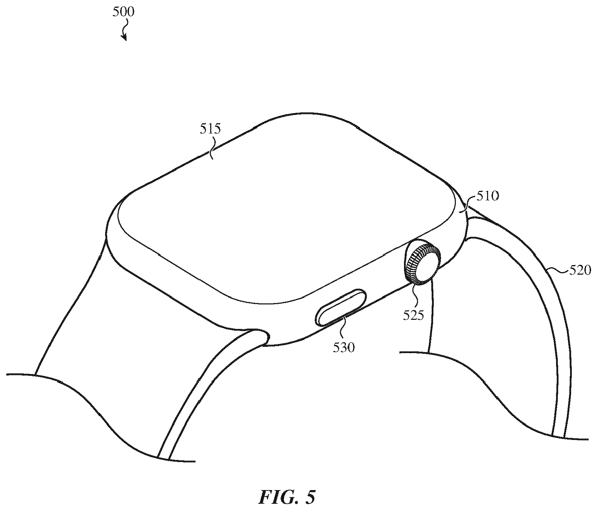

FIG. 5 illustrates a sample wearable device 500 that may execute the method 400 shown in FIG. 4 and/or incorporate the various modules of FIG. 2. Here, the wearable device 500 takes the form of a smart watch although other embodiments may be instantiated in other wearable devices, such as glasses, jewelry, clothing, and so on. Some embodiments may be portable electronic devices that are not necessarily wearable, such as smart phones, tablets, laptop computers, and the like.

In the example of FIG. 5, the wearable device 500 includes a housing 510 coupled to a display 515. The display 515 may show a user interface, such as calendar 300, to provide information regarding menstrual cycle estimates to a user. These may include estimates regarding a fertility window, period, ovulation, or the like. The display may be touch-sensitive, force-sensitive, or otherwise function as a user input. In this manner the display 515 may accept user data, for example for period tracking or similar.

The wearable device 500 may include one or more other user interfaces, such as rotatable crown 525, button 530, and the like. The user may rotate the crown, press or slide the button, or otherwise interact with these devices to provide input. As discussed herein, such input may be used to establish an initial day zero period/fertility window prediction, interact with a module such as the calendar module 200, or otherwise provide or request information related to menstrual cycle tracking.

The strap 520 may couple the wearable device 500 to the user. This may be especially useful where a heart rate sensor is integrated into or extends through part of the housing 510, for example a rear of the housing. The strap 520 may hold the wearable device in a position relative to the user that permits the heart rate sensor to operate unobtrusively. As one example, the strap may position the wearable device such that the heart rate sensor remains in continuous or near-continuous contact or proximity to the user. This may allow the heart rate sensor to gather heart rate data without requiring any prompts to the user.

As yet another option, a heart rate sensor may be integrated into the crown 525, button 530, display 515, or the strap 520.

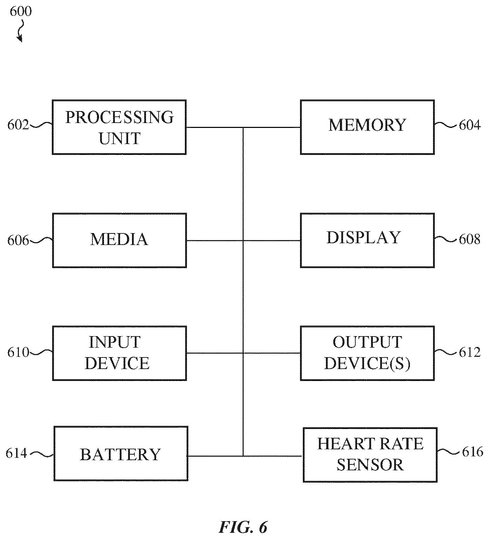

FIG. 6 depicts an example schematic diagram of an electronic device 600. By way of example, the device 600 of FIG. 6 may correspond to the wearable electronic device 500 shown in FIG. 5 (or any other wearable electronic device described herein). To the extent that multiple functionalities, operations, and structures are disclosed as being part of, incorporated into, or performed by the device 600, it should be understood that various embodiments may omit any or all such described functionalities, operations, and structures. Thus, different embodiments of the device 600 may have some, none, or all of the various capabilities, apparatuses, physical features, modes, and operating parameters discussed herein.

As shown in FIG. 6, a device 600 includes a processing unit 602 operatively connected to computer memory 604 and/or computer-readable media 606. The processing unit 602 may be operatively connected to the memory 604 and computer-readable media 606 components via an electronic bus or bridge. The processing unit 602 may include one or more computer processors or microcontrollers that are configured to perform operations in response to computer-readable instructions. The processing unit 602 may include the central processing unit (CPU) of the device. Additionally or alternatively, the processing unit 602 may include other processors within the device including application specific integrated chips (ASIC) and other microcontroller devices.

The memory 604 may include a variety of types of non-transitory computer-readable storage media, including, for example, read access memory (RAM), read-only memory (ROM), erasable programmable memory (e.g., EPROM and EEPROM), or flash memory. The memory 604 is configured to store computer-readable instructions, sensor values, and other persistent software elements. Computer-readable media 606 also includes a variety of types of non-transitory computer-readable storage media including, for example, a hard-drive storage device, a solid-state storage device, a portable magnetic storage device, or other similar device. The computer-readable media 606 may also be configured to store computer-readable instructions, sensor values, and other persistent software elements.

In this example, the processing unit 602 is operable to read computer-readable instructions stored on the memory 604 and/or computer-readable media 606. The computer-readable instructions may adapt the processing unit 602 to perform the operations or functions described above with respect to FIGS. 1-4. In particular, the processing unit 602, the memory 604, and/or the computer-readable media 606 may be configured to cooperate with an input device 610 (e.g., a rotation sensor that senses rotation of a crown component or a sensor that senses motion of a user's finger) to control the operation of a device in response to an input applied to a crown of a device (e.g., the crown 525). Likewise, the processing unit 602 may be configured to control and/or receive data from a sensor (e.g., heart rate sensor 616), as well as implement various operational modules to utilize that data to provide estimates of fertility window dates, ovulation dates, period dates, and/or other portions of a menstrual cycle. The computer-readable instructions may be provided as a computerprogram product, software application, or the like and may form the basis or source for any or all of the operational modules of FIG. 2.

As shown in FIG. 6, the device 600 also includes a display 608. The display 608 may include a liquid-crystal display (LCD), organic light emitting diode (OLED) display, light emitting diode (LED) display, or the like. If the display 608 is an LCD, the display 608 may also include a backlight component that can be controlled to provide variable levels of display brightness. If the display 608 is an OLED or LED type display, the brightness of the display 608 may be controlled by modifying the electrical signals that are provided to display elements. The display 608 may correspond to any of the displays shown or described herein.

The device 600 may also include a battery 614 that is configured to provide electrical power to the components of the device 600. The battery 614 may include one or more power storage cells that are linked together to provide an internal supply of electrical power. The battery 614 may be operatively coupled to power management circuitry that is configured to provide appropriate voltage and power levels for individual components or groups of components within the device 600. The battery 614, via power management circuitry, may be configured to receive power from an external source, such as an AC power outlet. The battery 614 may store received power so that the device 600 may operate without connection to an external power source for an extended period of time, which may range from several hours to several days.

In some embodiments, the device 600 includes one or more input devices 610. An input device 610 is a device that is configured to receive user input. The one or more input devices 610 may include, for example, a crown input system, a push button, a touch-activated button, a keyboard, a keypad, or the like (including any combination of these or other components). In some embodiments, the input device 610 may provide a dedicated or primary function, including, for example, a power button, volume buttons, home buttons, scroll wheels, and camera buttons.

The device 600 may also include a sensor 616, such as a heart rate sensor. The heart rate sensor 616 may monitor a user's heart rate when the device 600 is worn and generate signals indicative of the heart rate. The heart rate sensor 616 may operate continuously or at intervals, such as every five minutes. The resulting signals may be received by the processing unit 602 and used to estimate various portions of a menstrual cycle (such as period, fertility window, and/or ovulation) and/or stored in the memory 604 or media 606 for later use in this way. The heart rate sensor 600 may be an optical sensor, electrical sensor, or any other suitable sensor.

Additional functionality may be implemented by various embodiments beyond what is described above. For example, some embodiments may omit user logging/input of menstrual cycle data. For example, one or more sensors may be used to detect onset and termination of a period and not just ovulation. This may be done by measuring and correlating changes in a user's heart rate, temperature, or other biometric signal in a manner similar to that described herein with respect to ovulation. In such embodiments, user input may be unnecessary as the data gathered by the sensor may be sufficient to perform all estimations.

Certain embodiments may provide enhanced estimation of menstrual cycle portions (fertility windows, ovulation, periods) by clustering users. Data such as heart rate, temperature, or other biometric signals may be gathered and processed either locally or remotely to assign the user to a particular phenotype. Each phenotype has different details regarding menstrual cycles; people in one phenotype may have longer follicular phases than those in other phenotypes, shorter periods, less regular portion of a cycle, and so on. Some phenotypes may have certain commonalities that affect menstrual cycle details, such as being on common forms of birth control, taking certain hormones, common environmental factors, and the like. By using sensed biometric data to assign a user to a particular phenotype, the ovulation and period estimation modules may incorporate phenotype-specific data to more accurately estimate the dates and details of the user's menstrual cycle.

Certain embodiments may use multiple ovulation estimators and/or period estimators, each of which weights and/or analyzes data differently, and track how accurate each estimator is with respect to the user's menstrual cycle. Over time, the most accurate estimator may be used for future estimations, or a blend of multiple estimators may be used to more accurately create predictions.

Certain embodiments may split modules and/or functionality between multiple electronic devices, some of which may be remote from one another or otherwise connected to one another by a wired or wireless network. For example, a wearable electronic device may transmit heart rate data, calendar data, or both to a second electronic device that may perform ovulation estimates, fertility estimates, and transmit any estimate updates to the wearable electronic device. The second electronic device may be, for example, a smart phone, tablet, laptop computing device, remote server, and so on. Additionally, heart rate sensors may be incorporated in non-wearable devices. As one non-limiting example, a sleep monitor placed on or beneath a mattress may track a user's heart rate while they are sleeping as described herein. Further, multiple heart rate monitors incorporated into different electronic devices may be used to track heart rates; for example, the aforementioned sleep monitor may track a sleeping heart rate while a wearable device may track a waking heart rate. The data from each or all such devices may be collated by a second electronic device, a wearable device, or any other suitable electronic device and used to provide estimates of fertility windows, periods, and the like.

As described above, one aspect of the present technology is the gathering and use of data available from a user, such as heart rate data, menstrual cycle data, and the like. The present disclosure contemplates that in some instances, this gathered data may include personal information data that uniquely identifies or can be used to contact or locate a specific person. Such personal information data can include biometric data, demographic data, location-based data, telephone numbers, email addresses, Twitter IDs, home addresses, data or records relating to a user's health or level of fitness (e.g., vital signs measurements, medication information, exercise information), date of birth, or any other identifying or personal information. For example, where the sensor described above is a biometric sensor, sensitive and/or personal information may be captured about a user (e.g., health-related data).

The present disclosure recognizes that the use of such personal information data, in the present technology, can be used to the benefit of users. For example, the personal information data can be used to deliver targeted content that is of greater interest to the user. Accordingly, use of such personal information data enables users to calculated control of the delivered content. Further, other uses for personal information data that benefit the user are also contemplated by the present disclosure. For instance, health and fitness data may be used to provide insights into a user's general wellness, or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations. For instance, in the US, collection of or access to certain health data may be governed by federal and/or state laws, such as the Health Insurance Portability and Accountability Act (HIPAA); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of advertisement delivery services, the present technology can be configured to allow users to select to "opt in" or "opt out" of participation in the collection of personal information data during registration for services or anytime thereafter. In another example, users can select not to provide mood-associated data for targeted content delivery services. In yet another example, users can select to limit the length of time mood-associated data is maintained or entirely prohibit the development of a baseline mood profile. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, content can be selected and delivered to users by inferring preferences based on non-personal information data or a bare minimum amount of personal information, such as the content being requested by the device associated with a user, other non-personal information available to the content delivery services, or publicly available information.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

Exemplary embodiments of the present disclosure are set out in the following items:
1. A wearable device for estimating portions of a menstrual cycle, comprising:
   a calendar module;
   a heart rate sensor;
   a preprocessing module operative to:
      receive an initial period estimate from the calendar;
      receive heart rate data from the heart rate sensor; and
      process the initial period estimate and heart rate data into a processed data set;
   an ovulation estimator operative to:
      receive the processed data set from the preprocessing module; and
      use the processed data set to estimate a fertility window; and
   a period estimator operative to:
      receive the processed data set and an output from the ovulation estimator; and
      use the processed data set and the output from the ovulation estimator to estimate a period date.
2. The wearable device of item 1, further comprising:
   a fertile window update module configured to:
      receive the estimate of the fertility window from the ovulation estimator; and
      in the event the estimate of the fertility window starts in the future, provide the estimate of the fertility window to the calendar; and
   a period update module configured to:
      receive the estimate of the period from the period estimator; and
      in the event the estimate of the period starts in the future and the period has not been previously updated during a present menstrual cycle, provide the estimate of the period to the calendar.
3. The wearable device of item 2, wherein the calendar module is further operative to display the estimate of the fertility window and the estimate of the period to a user.
4. The wearable device of any of items 1-3, wherein the ovulation estimator is a combination of an LSTM neural network and a deep neural network.
5. The wearable device of any of items 1-4, wherein the initial period estimate comprises:
   an estimate of a period start date; and
   an estimate of a period end date.
6. A method for providing estimates of a fertile window, comprising:
   receiving an initial estimate of a fertile window and an initial estimate of a period;
   receiving heart rate data;
   determining whether the heart rate data covers a sufficient period of time;
   in the event the heart rate data covers the sufficient period of time, estimating an ovulation window using the heart rate data and the initial estimate of the fertile window; and
   in response to estimating the fertile window, updating a display of the fertile window that is electronically accessible by a user.
7. The method of item 6, further comprising:
   after estimating the fertile window, estimating a period using the heart rate data and the initial estimate of the period;
   in response to estimating the period, updating a display of the period that is electronically accessible by a user.
8. The method of any of items 6-7, wherein the sufficient period of time is at least half of a threshold number of preceding number of days.
9. The method of any of items 6-8, wherein the heart rate data comprises a basal sedentary waking heart rate.
10. The method of item 9, wherein the heart rate data further comprises a basal sedentary sleeping heart rate.
11. A system for estimating portions of a menstrual cycle, comprising:
   a wearable device comprising heart rate sensor;
   a calendar module;
   a preprocessing module operative to:
      receive an initial period estimate from the calendar;
      receive heart rate data from the heart rate sensor; and
      process the initial period estimate and heart rate data into a processed data set;
   an ovulation estimator operative to:
      receive the processed data set from the preprocessing module; and
      use the processed data set to estimate a fertility window; and
   a fertile window update module configured to:
      receive the estimate of the fertility window from the ovulation estimator; and
      in the event the estimate of the fertility window starts in the future, provide the estimate of the fertility window to the calendar.
12. The system of item 11, further comprising:
   a period estimator operative to:
   receive the processed data set and an output from the ovulation estimator; and
   use the processed data set and the output from the ovulation estimator to estimate a period date.
13. The system of item 12, further comprising:
   a period update module configured to:
   receive the estimate of the period from the period estimator; and
   in the event the estimate of the period starts in the future and the period has not been previously updated during a present menstrual cycle, provide the estimate of the period to the calendar.
14. The system of item 13, wherein the calendar module is further operative to display the estimate of the fertility window and the estimate of the period to a user.
15. The system of any of items 11-14, wherein the ovulation estimator is a combination of an LSTM neural network and a deep neural network.
16. The system of any of items 11-15, wherein the initial period estimate comprises:
   an estimate of a period start date; and
   an estimate of a period end date.
17. The system of any of items 11-16, wherein the preprocessing module is operative to determine whether the heart rate data covers a sufficient period of time prior to processing the initial period estimate and heart rate data into the processed data set.
18. The system of item 17, wherein the sufficient period of time is at least half of a threshold number of preceding number of days.
19. The system of any of items 11-18, wherein the heart rate data comprises a basal sedentary waking heart rate.
20. The system of item 19, wherein the heart rate data comprises a basal sedentary sleeping heart rate.

## Claims

1. A wearable device for estimating portions of a menstrual cycle, comprising:
a calendar module;
a heart rate sensor;
a preprocessing module operative to:
receive an initial period estimate from the calendar;
receive heart rate data from the heart rate sensor; and
process the initial period estimate and heart rate data into a processed data set;
an ovulation estimator operative to:
receive the processed data set from the preprocessing module; and
use the processed data set to estimate a fertility window; and
a period estimator operative to:
receive the processed data set and an output from the ovulation estimator; and
use the processed data set and the output from the ovulation estimator to estimate a period date.

2. The wearable device of claim 1, further comprising:
a fertile window update module configured to:
receive the estimate of the fertility window from the ovulation estimator; and
in the event the estimate of the fertility window starts in the future, provide the estimate of the fertility window to the calendar; and
a period update module configured to:
receive the estimate of the period from the period estimator; and
in the event the estimate of the period starts in the future and the period has not been previously updated during a present menstrual cycle, provide the estimate of the period to the calendar.

3. The wearable device of claim 2, wherein the calendar module is further operative to display the estimate of the fertility window and the estimate of the period to a user.

4. The wearable device of any of claims 1-3, wherein the ovulation estimator is a combination of an LSTM neural network and a deep neural network.

5. The wearable device of any of claims 1-4, wherein the initial period estimate comprises:
an estimate of a period start date; and
an estimate of a period end date.

6. A method for providing estimates of a fertile window, comprising:
receiving an initial estimate of a fertile window and an initial estimate of a period;
receiving heart rate data;
determining whether the heart rate data covers a sufficient period of time;
in the event the heart rate data covers the sufficient period of time, estimating an ovulation window using the heart rate data and the initial estimate of the fertile window; and
in response to estimating the fertile window, updating a display of the fertile window that is electronically accessible by a user.

7. The method of claim 6, further comprising:
after estimating the fertile window, estimating a period using the heart rate data and the initial estimate of the period;
in response to estimating the period, updating a display of the period that is electronically accessible by a user.

8. The method of any of claims 6-7, wherein the sufficient period of time is at least half of a threshold number of preceding number of days.

9. The method of any of claims 6-8, wherein the heart rate data comprises a basal sedentary waking heart rate.

10. The method of claim 9, wherein the heart rate data further comprises a basal sedentary sleeping heart rate.

11. A system for estimating portions of a menstrual cycle, comprising:
a wearable device comprising heart rate sensor;
a calendar module;
a preprocessing module operative to:
receive an initial period estimate from the calendar;
receive heart rate data from the heart rate sensor; and
process the initial period estimate and heart rate data into a processed data set;
an ovulation estimator operative to:
receive the processed data set from the preprocessing module; and
use the processed data set to estimate a fertility window; and
a fertile window update module configured to:
receive the estimate of the fertility window from the ovulation estimator; and
in the event the estimate of the fertility window starts in the future, provide the estimate of the fertility window to the calendar.

12. The system of claim 11, further comprising:
a period estimator operative to:
receive the processed data set and an output from the ovulation estimator; and
use the processed data set and the output from the ovulation estimator to estimate a period date.

13. The system of claim 12, further comprising:
a period update module configured to:
receive the estimate of the period from the period estimator; and
in the event the estimate of the period starts in the future and the period has not been previously updated during a present menstrual cycle, provide the estimate of the period to the calendar.

14. The system of claim 13, wherein the calendar module is further operative to display the estimate of the fertility window and the estimate of the period to a user.

15. The system of any of claims 11-14, wherein the initial period estimate comprises:
an estimate of a period start date; and
an estimate of a period end date.
